# EUROPEAN PATENT APPLICATION

(11) **EP 4 744 706 A1**
(43) Date of publication of application: **20.05.2026**
(21) Application number: 24840106.9
(22) Date of filing: 11.07.2024
(51) Int. Cl.: A61M 21/00, A61H 23/02

(54) **DEVICE FOR PREVENTING, ALLEVIATING, OR TREATING DEGENERATIVE BRAIN DISEASES, INDUCING BRAINWAVE SYNCHRONIZATION THROUGH TACTILE STIMULATION BY MEANS OF VIBRO-ACOUSTICS AND THROUGH MUSIC BY MEANS OF BONE CONDUCTION, AND METHOD THEREFOR**

(30) Priority: 11.07.2023 KR 20230089498
(71) Applicant: Aribio Co., Ltd., Seongnam-si, Gyeonggi-do 13535 (KR)
(72) Inventor: SHIN, Changho, Hwaseong-si Gyeonggi-do 18476 (KR); CHOE, Jaeyeong, Wonju-si Gangwon-do 26316 (KR); SUNG, Soohyun, Yangpyeong-gun Gyeonggi-do 12531 (KR); HA, Jae Young, Seongnam-si Gyeonggi-do 13595 (KR); CHOUNG, Jaijun, Seoul 06194 (KR)
(74) Representative: Vossius & Partner Patentanwälte Rechtsanwälte mbB
(86) International application number: PCT/KR2024/009926
(87) International publication number: WO 2025/014296

(57) **Abstract**

The present invention relates to: a device for preventing, alleviating, or treating degenerative brain diseases, the device inducing brainwave synchronization through tactile stimulation by means of vibro-acoustics and through music by means of bone conduction; and a method therefor.

Specifically, the present invention relates to a device capable of preventing, alleviating, or treating degenerative brain diseases (for example, mild cognitive impairment or early Alzheimer's disease (dementia)) and a method therefor, the device being worn on the head of a wearer so as to stimulate the brain by providing vibro-acoustics consisting of 40 Hz vibration and sound to the frontal lobe and temporal lobe of the wearer, thereby normalizing cerebral gamma oscillations caused by increased cerebral blood flow.

## Description

### [TECHNICAL FIELD]

The present invention relates to a device and method for preventing, alleviating, or treating degenerative brain diseases by inducing brainwave synchronization through tactile stimulation by acoustic vibration and music by bone conduction.

Specifically, the present invention relates to a device and method for preventing, alleviating, or treating degenerative brain diseases (such as mild cognitive impairment or early Alzheimer's disease (dementia)) by stimulating the brain by providing acoustic vibration (vibro-acoustics) consisting of low-frequency (30 Hz to 120 Hz) vibration and sound passing through a wearer's skull while being worn on the wearer's head, thereby normalizing cerebral gamma oscillation due to increased cerebral blood flow.

### [BACKGROUND ART]

The human body naturally ages over time, which causes natural pain and limits activity due to damage caused by partial degeneration. In addition, various diseases such as brain damage, myocardial infarction, arteriosclerosis, and arthritis are occurring due to restrictions on activities caused by industrial and technological development, traffic accidents, industrial accidents, sports injuries, simple accidents during activities, and various complications caused by stress, lack of exercise, and obesity.

To treat pain, injury, obesity, and the like caused by such natural human phenomena or socio-cultural problems, various stimulating devices are being developed and utilized along with pharmaceuticals and health functional foods. These stimulation devices include a massage device.

Generally, the massage device is a device that stimulates the skin or scalp by tapping or rubbing the same to promote smooth blood circulation and fat decomposition and massages to discharge waste. Such a massage device generates vibration or electrical stimulation using an electrical signal and applies the same to the skin or scalp. For example, the massage devices include a low-frequency massage device that massages by attaching an electrode to a surface of the skin to allow a low-frequency current to pass through the human body, an ultrasonic massage device that transmits ultrasonic vibration to the human body by bringing a probe for ultrasonic irradiation into contact with the surface of the skin, and a massage device using ultra-low frequency, far-infrared rays, and the like.

However, human body vibration stimulation technologies currently being utilized include linear stimulation using a simple rotary vibration motor and a solenoid, a pressurization method using air pressure, a stimulator inducing muscle contraction by inputting a low-frequency current, a method of contracting muscles using a magnetic field, and the like, but they are limited to simply applying pressure or stimulation to the human body and each technology has limitations.

For example, a vibrator using a motor has a disadvantage in that a frequency thereof can be adjusted, but an amplitude or intensity thereof cannot be adjusted, causing damage to the human body due to incorrect use, and has a structure and characteristics that fail to transmit the frequency rhythmically.

Recently, stimulation methods utilizing the principle of a speaker have been provided to supplement this, but they do not implement an intensity sufficient for a smooth experience and merely perform a function of a subwoofer of a speaker.

In addition, a technology utilizing a low-frequency current, a technology utilizing ultrasonic waves, a technology utilizing a high frequency, and the like have been disclosed and applied, but the low frequency causes much inconvenience to a user, the ultrasonic waves are difficult for the user to immediately adapt to a feeling and effect thereof, and there is a fatal difficulty in use in that an affected part is exposed and a medium for transmission of the low frequency or the ultrasonic waves is required.

Since the high frequency aims at deep heat rather than being a stimulation device, a risk factor for the user is inherent due to the characteristics of the high frequency along with user anxiety. In the case of the high frequency, a medium for exposure and transmission of the affected part is required in use, and a conductive plate for transmission of an anode of electricity is utilized, which is dangerous and causes much inconvenience in use.

Since a low-frequency treatment device continuously and repeatedly applies a low-frequency current in the form of a low-frequency pulse to the skin through an electrode, it gives a feeling of electric shock, causing an unpleasant feeling during treatment, which reduces a treatment effect. In addition, the low-frequency treatment device has a problem that women avoid the device because the affected part must be exposed to attach the electrode to the skin.

In addition, in an ultrasonic treatment and beauty device, ultrasonic vibration is propagated when a skin contact surface of a probe for ultrasonic irradiation comes into contact with the skin, but if a method of applying the probe to the skin is incorrect, the ultrasonic vibration is not propagated, so that a sufficient effect cannot be obtained. Regardless of contact or non-contact of the probe with the skin, ultrasonic waves of an output value set by the user are irradiated. Therefore, during non-contact, a vibration propagation part of the probe generates heat due to vibration, and a temperature of this part rises, giving discomfort to the user, and there are various problems such as a risk of burns when used for a long time.

In addition, various skin care modes can be implemented using a vibrator that vibrates in a vertical direction by a magnetic coil method, and various technologies capable of galvanic massage and iontophoresis massage using the vibrator are disclosed.

However, since such a conventional technology applies a method of stimulating the skin by converting a rotational force of a vibration motor into a linear motion or a cam motion, a large noise is generated in a process of transmitting power of the vibration motor, giving discomfort to users of the beauty device. Since it is a principle of generating vibration by eccentricity, a force distributed in a horizontal direction, that is, a direction parallel to a skin surface is large, and a force acting perpendicularly to the skin surface is small, so that there is a problem that skin massage cannot be effectively performed.

In addition, a device for generating sound waves by applying the principle of a speaker and a device capable of generating a sound source have been developed, but there are problems such as a small frequency generation width and very weak intensity due to problems such as structural characteristics of a magnetic circuit and positions of a leaf spring and a coil. In addition, since a guide utilizing a bearing for maintaining acoustic vibration in a vertical form and a coil spring for maintaining elasticity must be configured, there is a limit to miniaturization.

Such a conventional technology has a limitation in effectively managing the human body because use modes and functions are very limited.

### [SUMMARY OF THE INVENTION]

### [TECHNICAL PROBLEM]

An object of the present invention is to provide a device and method for preventing, alleviating, or treating degenerative brain diseases (such as mild cognitive impairment or early Alzheimer's disease (dementia)) by normalizing cerebral gamma oscillation due to increased cerebral blood flow by stimulating the brain by providing acoustic vibration (vibro-acoustics) composed of 40 Hz vibration and sound to a frontal lobe and a temporal lobe of a wearer while being worn on the head of the wearer.

### [TECHNICAL SOLUTION]

In order to achieve the above object, a device for preventing, alleviating, or treating degenerative brain diseases by inducing brainwave synchronization through tactile stimulation by acoustic vibration and music by bone conduction according to the present invention is a device worn on a wearer's head to induce brainwave synchronization through tactile stimulation by acoustic vibration and music by bone conduction, wherein the device includes contact portions provided in each of two directions corresponding to a temporal lobe of the wearer and one direction corresponding to a frontal lobe of the wearer to provide acoustic vibration to the temporal lobe and the frontal lobe of the wearer.

### [ADVANTAGEOUS EFFECTS]

According to the device and method for preventing, alleviating, or treating degenerative brain diseases by inducing brainwave synchronization through tactile stimulation by acoustic vibration and music by bone conduction according to the present invention, by being worn on the head of the wearer and providing acoustic vibration (vibro-acoustics) composed of 40 Hz vibration and sound to the frontal lobe and the temporal lobe of the wearer to stimulate the brain, thereby normalizing cerebral gamma oscillation due to increased cerebral blood flow, there is an effect of achieving prevention, alleviation, or treatment of degenerative brain diseases (such as mild cognitive impairment or early Alzheimer's disease (dementia)).

### [BRIEF DESCRIPTION OF DRAWINGS]

FIGS. 1 to 3 are views respectively showing a vibration device used in the device for preventing, alleviating, or treating degenerative brain diseases by inducing brainwave synchronization through tactile stimulation by acoustic vibration and music by bone conduction according to the present invention from multiple directions.
FIG. 4 is an exploded perspective view showing the vibration device used in the device for preventing, alleviating, or treating degenerative brain diseases by inducing brainwave synchronization through tactile stimulation by acoustic vibration and music by bone conduction according to the present invention.
FIG. 5 shows a coupled state of the vibration device of FIG. 4.
FIG. 5A shows another example of the vibration device.
FIG. 5B is an enlarged view of the elastic body of FIG. 5A.
FIGS. 6A and 6B show a coupled state according to another embodiment of the connection member of FIG. 4.

### [MODES FOR CARRYING OUT THE INVENTION]

Terms or words used in the present specification and claims should not be construed as being limited to ordinary or dictionary meanings, and should be construed as meanings and concepts consistent with the technical idea of the present invention based on the principle that the inventor can appropriately define concepts of terms to explain his or her invention in the best way.

Therefore, the embodiments described in the present specification and the configurations shown in the drawings are merely the most preferred embodiments of the present invention and do not represent all the technical ideas of the present invention, so it should be understood that there may be various equivalents and modifications that can replace them at the time of the present application.

Hereinafter, prior to description with reference to the drawings, it is clarified that matters not necessary to reveal the gist of the present invention, that is, known configurations that can be obviously added by those skilled in the art, are not shown or described in detail.

The present invention relates to a device and method for preventing, alleviating, or treating degenerative brain diseases by inducing brainwave synchronization through tactile stimulation by acoustic vibration and music by bone conduction.

Specifically, the present invention relates to a device and method for preventing, alleviating, or treating degenerative brain diseases (such as mild cognitive impairment or early Alzheimer's disease (dementia)) by stimulating the brain by providing acoustic vibration (vibro-acoustics) consisting of low-frequency (30 Hz to 120 Hz) vibration and sound passing through a wearer's skull while being worn on the wearer's head, thereby normalizing cerebral gamma oscillation due to increased cerebral blood flow.

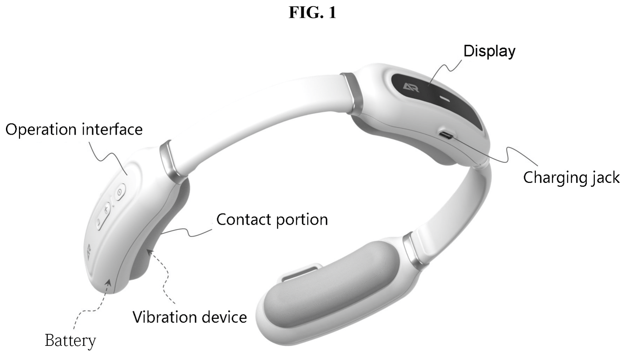

FIGS. 1 to 3 are views respectively showing a vibration device used in the device for preventing, alleviating, or treating degenerative brain diseases by inducing brainwave synchronization through tactile stimulation by acoustic vibration and music by bone conduction according to the present invention from multiple directions.

The device for preventing, alleviating, or treating degenerative brain diseases by inducing brainwave synchronization through tactile stimulation by acoustic vibration and music by bone conduction according to the present invention according to the accompanying drawings has a ring shape so as to be wrapped around the wearer's head, one side of which is cut to facilitate wearing, and elasticity is imparted to the ring shape to improve wearing comfort.

In addition, the device according to the present invention is configured to provide acoustic vibration in a total of three directions. Specifically, due to the ring shape, respective ends are positioned at the temporal lobes of the wearer, and one side is positioned at the frontal lobe of the wearer, so that a total of three contact portions are provided.

The contact portion is configured in a form of a cover made of a flexible material on a surface thereof, and a vibration device 100 is coupled therein to generate acoustic vibration.

The vibration device 100 is as described below.

In addition, a battery for power supply is provided inside the contact portion together with the vibration device 100, and an operation interface including a power button for On/Off and a button for adjusting an intensity of the acoustic vibration may be provided on one side of an outer surface of the device.

In addition, a charging jack for charging power required for the device and supplying the power to the battery, and a display for displaying an operating state of the device may be provided on the other side of the device.

The device configured as described above may be provided with a separate database to store sound sources, but according to an embodiment, may communicate with a terminal to receive sound source information.

Hereinafter, Vibroacoustic Stimulation will be described in detail.

This is to provide acoustic vibration by combining 40 Hz vibration and music to induce gamma wave synchronization, and has a wavelength when 40 Hz is combined with music (sound source) as shown in [Table 1].

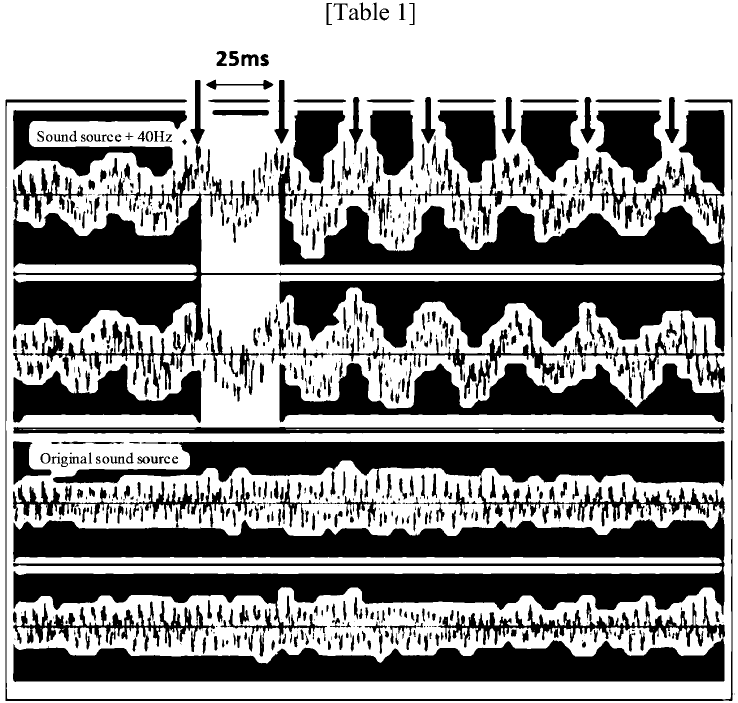

When such acoustic vibration is provided tactually through vibration, the amount of Cerebral Blood Flow (CBF) increases, and Oxyhemoglobin (HbO2) increases to activate nerve cells.

When the acoustic vibration is provided auditorily through a sound source, the auditory cortex and amygdala are stimulated to induce autobiographical memory recall. Since both of these are provided tactually and auditorily, memory and cognitive ability can be improved as shown in [Table 2], thereby achieving prevention, alleviation, or treatment of degenerative brain diseases (e.g., mild cognitive impairment or early Alzheimer's disease (dementia)).

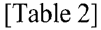

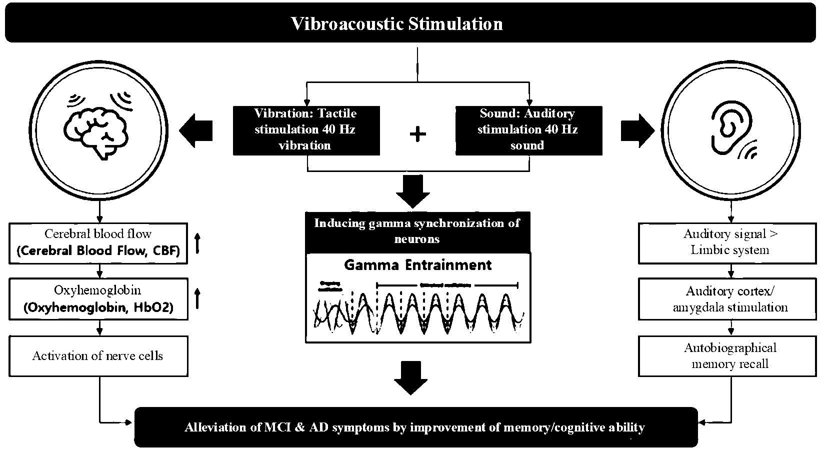

Regarding stimulation methods conventionally used for the purpose of brain diseases, there are Deep Brain Stimulation (DBS), Transcranial Alternating Current/Direct Current Stimulation (tACS, tDCS), Transcranial Magnetic Stimulation (TMS), and Ultrasound stimulation methods, which are as shown in [Table 3].

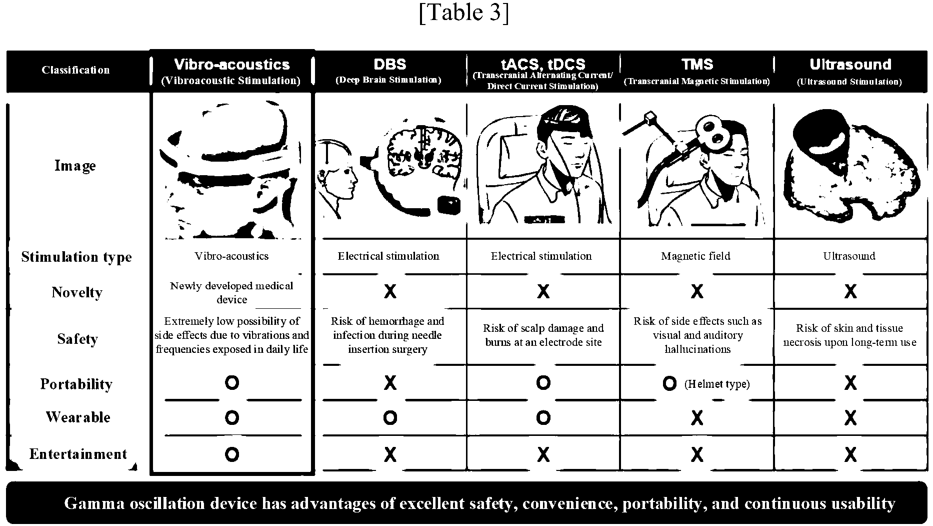

That is, in the case of Deep Brain Stimulation (DBS), in addition to the risk of needle insertion, there is a very high risk of hemorrhage and infection during surgery, and in the case of Transcranial Alternating Current/Direct Current Stimulation (tACS, tDCS), there is a risk of scalp damage at the electrode site as well as a risk of burns.

In addition, in the case of Transcranial Magnetic Stimulation (TMS), there is a risk of side effects such as visual and auditory hallucinations, and in the case of Ultrasound Stimulation, there is a risk of skin and tissue necrosis due to long-term use.

On the other hand, according to the vibroacoustic stimulation of the present invention, since only 40 Hz, which is a vibration and frequency exposed in daily life, is used, the possibility of side effects can be seen as very low.

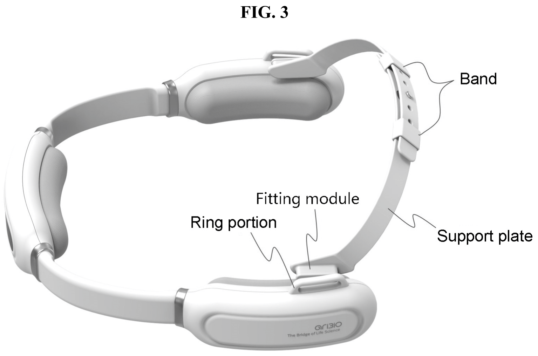

As shown in FIG. 3 of the accompanying drawings, the device according to the present invention is provided with a ring portion at each end of the device, and a fitting module is coupled through the ring portion.

The fitting module extends by being coupled with a support plate having predetermined flexibility, and since there are two fitting modules coupled to the ring portions provided at each end, the support plates are also composed of two.

At this time, one of the respective support plates is provided with a plurality of arranged through-holes, and the other one is provided with protrusions, so that when the two support plates are adjacent to face each other, the protrusions provided on the one support plate are inserted into and coupled with the through-holes provided on the other support plate, thereby allowing the two support plates to be coupled.

In addition, to maintain a coupling force, the two support plates are stably coupled using a band.

In addition, the support plate integrally extending from the fitting module has a predetermined angle, and due to this angle, the support plate is positioned on the scalp of the wearer's head, thereby enabling more stable wearing of the device.

Also, by being coupled to the ring portion through the fitting module, when the fitting module is detached from the ring portion, the device may be worn excluding the support plate.

Hereinafter, the vibration device 100 will be described in detail.

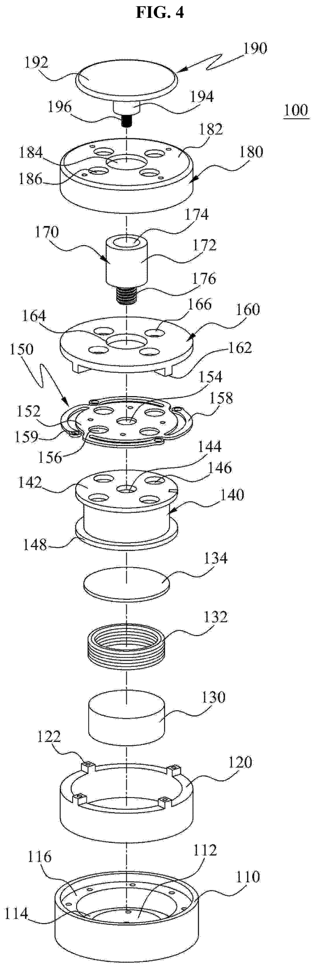

The vibration device (100) according to the present invention is described with reference to FIG. 4 and below of the attached drawings.

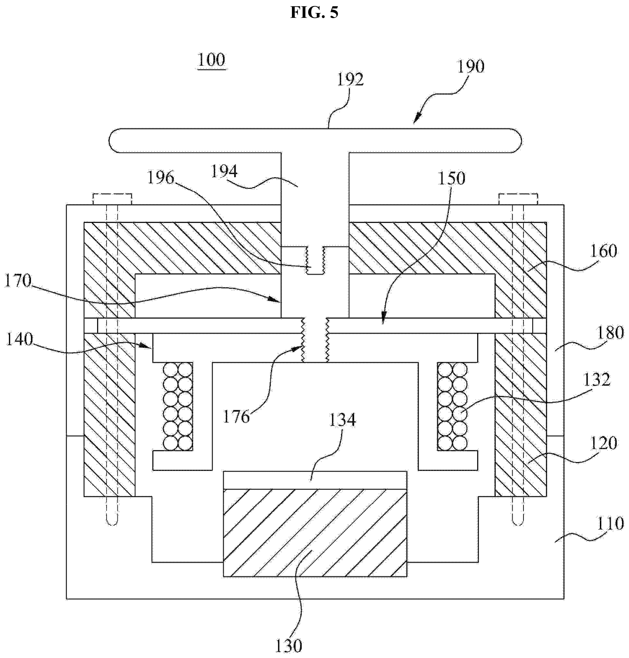

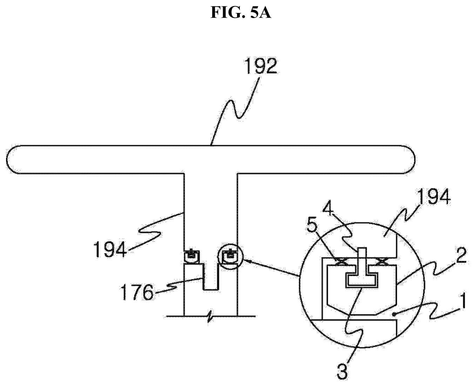

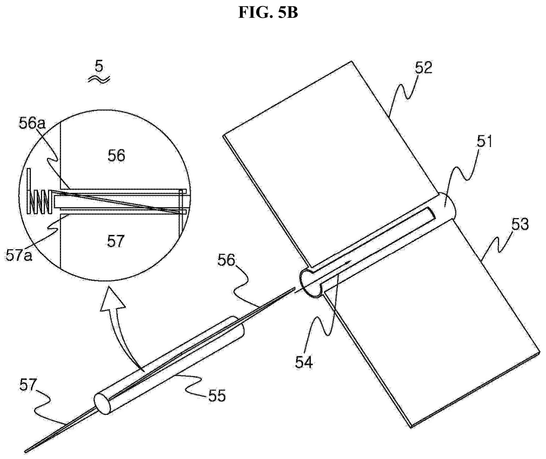

Referring to FIGS. 4 and 5, the vibration device 100 of the present invention includes upper and lower bodies 180 and 110, upper and lower brackets 160 and 120, a magnetic body 130, a voice coil 132, an upper plate 134, a bobbin 140, a cone damper 150, and a connecting member 170. In addition, although not shown in the drawings, the vibration device 100 may be further provided with a waterproof member or a buffer member.

In this embodiment, the configuration of the vibration device will be described in detail using the connecting member 170 for transmitting vibration and a vibration probe 190 coupled to the connecting member 170 to massage and stimulate the human body, in order to transmit vibration generated from the vibration device 100 to the outside so as to massage and stimulate the skin or scalp of the human body.

That is, the vibration probe 190 is detachably provided in the vibration device 100.

Such a vibration probe 190 may have various shapes and sizes to be suitable for various uses of a human body stimulation device (200 in FIG. 4) to be described later.

The vibration probe 190 of this embodiment includes a plate 192 on which a massage or stimulation head (not shown) is mounted and detached, a shaft 194 coupled to the center of a lower surface of the plate 192, and a coupling portion 196 provided at a lower end of the shaft 194 and coupled to the connecting member 170. Here, the coupling portion 196 is provided in a form of a bolt screwed to the connecting member 170.

Meanwhile, the above-described vibration probe 190 has an upwardly concave groove portion 1 formed on one side of a lower surface of the shaft 194 where the bolt 176 is not formed, and a vibrating body 2 is coupled to the groove portion 1 by a connecting body 4.

At this time, the connecting body 4 has a two-stage shape of 'convex', and a part of the connecting body 4 is inserted into and fixed to an insertion groove 3 formed in the vibrating body 2.

The size of the insertion groove 3 is made larger than the size of the part of the connecting body 4 inserted into the insertion groove.

This is shown in FIG. 5A.

FIG. 5A shows another example of the vibration device.

In addition, the vibrating body 2 has a smaller size than the groove portion 1 so that it can swing around the connecting body 4 within the groove portion 1.

At this time, an elastic body 5 is provided between one surface of the groove portion 1 and an upper surface of the vibrating body 2, and the above-described elastic body 5 can be referred to FIG. 5B of the accompanying drawings.

FIG. 5B is an enlarged view of the elastic body of FIG. 5A.

The elastic body 5 according to FIG. 5B of the accompanying drawings includes a first shaft 51 having a cylindrical shape with a hollow inside and having a first wing 52 and a second wing 53 extending symmetrically to both sides, and including two guide grooves 54 formed along a longitudinal direction on one side and having one end open; and a second shaft 55 having a cylindrical shape and having a third wing 56 and a fourth wing 57 extending symmetrically to both sides, wherein the third wing 56 and the fourth wing 57 respectively include insertion grooves 56a and 57a formed to have a predetermined length adjacent to the second shaft 55.

That is, the second shaft 55 is inserted into the hollow of the first shaft 51 as the third wing 56 and the fourth wing 57 are inserted into and guided by the respective guide grooves 54 of the first shaft 51.

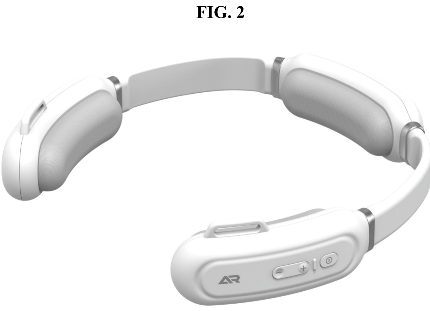

In addition, a torsion spring is inserted through the insertion grooves 56a and 57a as shown in FIG. 2B. At this time, one protruding end of the torsion spring is closely fixed to one surface of the third wing 56, and the other protruding end in the other direction is closely fixed to one surface of the second wing 53.

Accordingly, the elastic body 50 has an 'X' shape due to the elastic force of the torsion spring, and can be driven to fold within a range in which the third wing 56 and the fourth wing 57 can move from the guide grooves 54.

With this configuration, the intensity of vibration of the shaft 194 of the vibration probe 190 according to the present invention is assisted and further increased by the vibrating body 2, and the impact due to vibration applied to the probe itself is reduced even if the vibration intensity is increased by the elastic body 5.

At this time, since the vibrating body 2 includes an inclined surface inclined in a predetermined direction on a lower surface thereof as shown in FIG. 2a of the accompanying drawings, the vibrating body 2 can swing without being affected by an upper surface of the connecting member 170 corresponding to a lower surface of the groove portion 1. Examples of such a lower surface may have a wedge or cone shape, unlike the accompanying drawings.

Specifically, the lower body 110 is provided in a cylindrical shape with an open top and forms a space in which the magnetic body 130 is installed. The lower body 110 has the magnetic body 130 fixedly installed on a bottom surface of the internal space. In addition, the lower bracket 120 is inserted and installed in the lower body 110 outside the magnetic body 130 in the internal space. To this end, the lower body 110 includes a fixing groove 112 formed on the bottom surface where the magnetic body 130 is fixedly installed, a ring-shaped separation groove 114 provided to be spaced apart from an outer circumferential surface of the magnetic body 130 fixed to the fixing groove 112 by a predetermined distance, and a ring-shaped mounting groove 116 where the lower bracket 120 is mounted to be spaced apart from the magnetic body 130 by a predetermined distance. The separation groove 114 is provided to form a magnetic path for a magnetic field formed by the magnetic body 130 and the voice coil 132.

The upper body 180 is provided in a cylindrical shape with an open bottom and covers the open top of the lower body 110. The upper body 180 is coupled to the lower body 110 to form a space for accommodating the components 120 to 160 therein. The upper surface 182 of the upper body 180 is formed with an insertion hole 184 in the center through which the connecting member 170 and the shaft 194 of the vibration probe 190 are inserted, and a plurality of heat dissipation holes 186 provided outside the insertion hole 184 to dissipate heat generated when vibration occurs in the vibration device 100. In addition, a waterproof member (not shown) such as silicon may be further provided on the upper surface 182 of the upper body 180 for waterproofing the insertion hole 184 into which the vibration probe 190 is inserted. These upper body 180 and lower body 110 are made of aluminum material to improve heat dissipation effect.

The lower bracket 120 is provided in a cylindrical shape with open top and bottom. The lower bracket 120 is installed in the mounting groove 116 of the lower body 110, and an upper surface thereof is coupled to the cone damper 150. To this end, the lower bracket 120 is provided with a plurality of coupling protrusions 122 on the upper surface. The bobbin 140 mounted with the voice coil 132 is installed inside the lower bracket 120. A part of the lower bracket 120 protrudes to the upper part of the lower body 110. When the lower bracket 120 is coupled to the damper 158 of the cone damper 150 at the coupling protrusion 122, it may be fixed by further using a silicon washer or the like to ensure durability and maintain vibration force. This allows selective use of silicon washers to adjust the height of the cone damper 150 according to frequency characteristics, thereby performing a function for efficient amplitude maintenance.

The upper bracket 160 is provided in a plate shape to be accommodated inside the upper body 180, and an edge thereof is coupled to the upper part of the lower bracket 120. A plurality of coupling protrusions 162 are provided on a lower surface of the edge of the upper bracket 160 so that the cone damper 150 and the lower bracket 120 are coupled.

The upper surface of the upper bracket 160 is generally circular facing the lower bracket 120. An insertion hole 164 through which the connecting member 170 is inserted is formed in the center of the upper surface of the upper bracket 160, and a plurality of heat dissipation holes 166 are provided outside the insertion hole 164 to dissipate heat generated when vibration occurs in the vibration device 100. The insertion holes 184 and 164 of the upper body 180 and the upper bracket 160 fix the connecting member 170 and the vibration probe 190 to the center of the vibration device 100. In addition, a buffer member (not shown) for preventing unnecessary vibration transmission, such as a leaf spring, may be further provided between the upper surface of the upper bracket 160 and the upper body 180.

The magnetic body 130 is fixedly installed in the fixing groove 112 of the lower body 110 and interacts with the voice coil 132 to generate a magnetic field. The magnetic body 130 is provided as a ferromagnetic permanent magnet such as a neodymium magnet. The upper plate 134 is installed on the upper surface of the magnetic body 130.

The voice coil 132 is installed on an outer circumferential surface of the bobbin 140 at an upper portion of the magnetic body 130. The voice coil 132 is guided by the bobbin 140 to be stably installed. The voice coil 132 receives power and interacts with the magnetic body 130 to generate a magnetic field. The magnetic body 130 and the voice coil 132 are installed inside the lower bracket 120.

The upper plate 134 has a shape substantially similar to an upper surface of the magnetic body 130, is provided on an upper portion of the magnetic body 130, and is installed to be adjacent to a lower surface of the bobbin 140. The upper plate 134 induces magnetic force of the magnetic body 130 to be concentrated on the voice coil 132 in order to prevent loss of a magnetic field generated by the magnetic body 130. A magnetic fluid (not shown) may be applied to an outer diameter portion of the upper plate 134 to form a magnetic field.

The bobbin 140 is made of a non-magnetic material, such as an aluminum material. The bobbin 140 is installed inside the lower bracket 120. The bobbin 140 guides the voice coil 132 to be stably installed on the outside, thereby preventing the voice coil 132 from being separated. The bobbin 140 is coupled to the connecting member 170 at the center of an upper surface thereof. To this end, the bobbin 140 is provided in a cylindrical shape having open top and bottom sides, wherein an upper surface 142 is larger than a radius of the side surface, and a lower portion of the side surface extends outward to have a lower surface 148 facing the upper surface 142. The voice coil 132 is installed on the side surface of the bobbin 140. At this time, the voice coil 132 is guided by the upper surface 142 and the lower surface 148 of the bobbin 140.

In addition, on the upper surface 142 of the bobbin 140, a coupling hole 144 coupled to a lower end of the connecting member 170 is formed in the center, and a plurality of heat dissipation holes 146 provided outside the coupling hole 144 to dissipate heat generated when vibration occurs in the vibration device 100 are formed. These heat dissipation holes 146 function to reduce noise when vibration occurs, along with a heat dissipation effect. In addition, the bobbin 140 dissipates heat generated from the voice coil 132.

The bobbin 140 creates an efficient magnetic field as the magnetic body 130 is fixed to the fixing groove 112 of the lower body 110 and located at an inner diameter portion of the lower surface 148 of the bobbin 140, thereby generating mutual attraction and repulsion during magnetization of the voice coil 132 wound around the bobbin 140 to generate a stable vibration force. In addition, since the vibration probe 190 is coupled to the bobbin 140 through the connecting member 170, the bobbin 140 serves to hold eccentricity by a strong magnetic path generated by the combination of the magnetic body 130 and the upper plate 134 even if physical eccentricity occurs during human body stimulation by the vibration probe 190.

The cone damper 150 is installed on the upper surface 142 of the bobbin 140 and generates vibration in a vertical direction by using a magnetic field generated by interaction between the magnetic body 130 and the voice coil 132. That is, the cone damper 150 generates vibration by acting like a speaker for sound of a sound source as vibration caused by the sound source acts as vibration of air. The cone damper 150 is coupled to the upper and lower brackets 120 and 160 at an edge thereof. To this end, the cone damper 150 includes a cone plate 152 and a plurality of dampers 158.

The cone plate 152 has a coupling hole 154 formed in the center to be coupled to the lower end of the connecting member 170, and a plurality of heat dissipation holes 156 are formed outside the coupling hole 154. Each of the dampers 158 is provided in a shape extending long in a radially curved band shape of the cone plate 152 in order to maximize vibration force of the cone damper 150, and a coupling hole 159 for screw coupling is formed at an end thereof. The dampers 158 are screw-coupled and fixed between the coupling protrusions 162 of the upper bracket 160 and the coupling protrusions 122 of the lower bracket 120 through the coupling holes 159.

In addition, in order to ensure durability, the cone damper 150 is supported by using elastic members such as silicone washers at upper and lower portions of the coupling holes 159 of the dampers 158, thereby preventing vibration from being attenuated when vibration occurs and generating soft sound.

Therefore, the cone damper 150 generates vibration in response to a change in acoustic pressure of a sound source supplied from the outside. The cone damper 150 has the connecting member 170 coupled to a central portion thereof, and transmits vibration to the outside, for example, the vibration probe 190, through the connecting member 170. Since the central portion of the cone damper 150 has the largest amplitude of vibration, vibration transmission efficiency can be improved by coupling the bolt 176 of the connecting member 170 and the coupling hole 154 of the cone damper 150. In this embodiment, in the vibration device 100, the connecting member 170 is coupled to the center of the upper surface 142 of the cone damper 150 and the bobbin 140 so that coupling force with the connecting member 170 is improved.

And the connecting member 170 directly receives the vibration generated from the cone damper 150 and transmits the vibration to the outside. In this embodiment, the connecting member 170 is coupled to the shaft 194 of the vibration probe 190. That is, the connecting member 170 is provided in a shaft shape, an upper portion thereof is coupled to the shaft 194 of the vibration probe 190, and a lower portion thereof is coupled to the cone damper 150 and the upper surface 142 of the bobbin 140.

To this end, the connecting member 170 includes a shaft-shaped body 172, a coupling groove 174 in which the shaft 194 is coupled to an upper portion of the body 172, and a coupling bolt 176 coupled to the coupling hole 154 of the cone damper 150 and the coupling hole 144 of the bobbin 140 at a lower portion of the body 172. In this embodiment, the inside of the coupling groove 174 has a structure in which the coupling portion 196 of the shaft 194 is screw-coupled.

Therefore, in the vibration device 100 of the present invention, the shaft 194 of the vibration probe 190 is inserted through the center of the upper body 180 and coupled to the connecting member 170, and the connecting member 170 is inserted through the upper bracket 160 and screw-coupled to the central portion of the cone damper 150 and the bobbin 140. Accordingly, in the vibration device 100, when the cone damper 150 generates vibration in the vertical direction due to acoustic pressure, the vibration is transmitted to the vibration probe 190 through the connecting member 170 coupled to the cone damper 150.

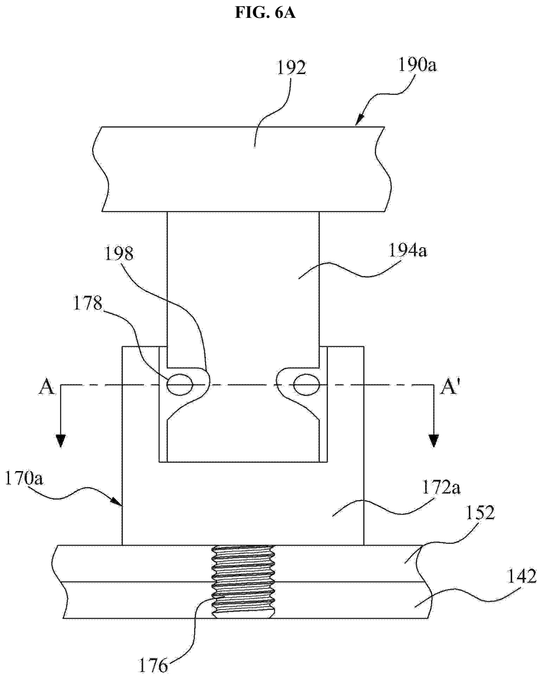

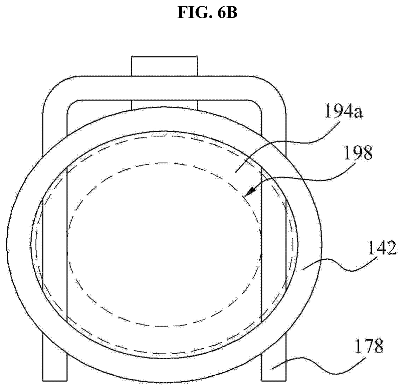

Another embodiment of the connecting member 170 is shown in FIG. 6.

Referring to FIGS. 6A and 6B, in this embodiment, a vibration probe 190a has a fixing groove 198 formed at a specific position along an outer circumferential surface of a shaft 194a. The fixing groove 198 is formed, for example, on an outer circumferential surface of a portion where the shaft 194a of the vibration probe 190a is inserted into the coupling groove 174 of the connecting member 170a. The fixing groove 125 has a catch formed at an upper portion and a guide portion formed at a lower portion.

And, an elastic fixing pin 178 that is inserted into and detached from the fixing groove 198 is fixedly mounted on the connecting member 170a. The elastic fixing pin 178 is fixedly coupled to the outside of the connecting member 170a, a part thereof is exposed inside the coupling groove 174 of the connecting member 170a, and the exposed part is seated in or separated from the fixing groove 198 of the shaft 194a.

When the shaft 194a of the vibration probe 190a is pressed downward and inserted into the coupling groove 174, the connecting member 170a is guided so that the elastic fixing pin 178 is seated in the fixing groove 198 by the guide portion of the fixing groove 198, and at the same time, the elastic fixing pin 178 is fixedly mounted by the catch. In addition, when the shaft 194a is pressed upward and separated from the coupling groove 174, the elastic fixing pin 178 is easily separated from the fixing groove 178 by the guide portion of the fixing groove 198.

In the case of this embodiment, the vibration probe 190a can be more easily coupled to and separated from the connecting member 170 than the screw coupling structure of FIGS. 4 and 5, and damage to the coupling portion 196 of the vibration probe 190 that may occur as the vibration probe 190a is coupled to and separated from the vibration device 100 multiple times can be prevented.

Therefore, since the vibration probes 190 and 190a of the present invention use the connecting members 170 and 170a coupled using screw coupling or the fixing groove 198 and the elastic fixing pin 178, assembly is easy during manufacturing.

As described above, the vibration device 100 of the present invention couples the connecting members 170 and 170a and the shafts 194 and 194a of the vibration probes 190 and 190a to the central portion of the cone damper 150 generating vibration in response to a change in acoustic pressure of a sound source, thereby directly receiving vibration from the central portion of the cone damper 150. In addition, the vibration device 100 of the present invention couples the connecting member 170 and the shafts 194 and 194a of the vibration probes 190 and 190a together with the cone damper 150 to the center of the upper surface 142 of the bobbin 140, thereby improving coupling force with the connecting member and improving vibration transmission efficiency through this.

In addition, the vibration device 100 of the present invention can improve vibration generation efficiency by configuring the cone damper 150 in a leaf spring shape with a cone plate 152 having a sound wave generating function and dampers 158 having vibration force transmission and spring functions, in order to generate stable vibration.

In addition, although the vibration device 100 of the present invention is described as having a structure including upper and lower bodies 110 and 180 and upper and lower brackets 120 and 160, it may be implemented in a structure without the upper body 180 and the upper bracket 160 in order to further simplify the structure and improve vibration generation efficiency and heat dissipation effect. This is because the connection with the vibration probe 190 is strong since the connecting member 170 is coupled to the cone damper 150 and the upper surface 142 of the bobbin 140.

The above description using the drawings describes only the main points of the present invention, and it is obvious that the present invention is not limited to the configuration of the drawings, as various designs are possible within the technical scope.

## Claims

1. A device for inducing brainwave synchronization through tactile stimulation by acoustic vibration and music by bone conduction, worn on a head of a wearer, wherein
the device comprises:
contact portions provided in respective ones of two directions corresponding to temporal lobes of the wearer and one direction corresponding to a frontal lobe of the wearer; and wherein
the contact portions provide acoustic vibration to the temporal lobes and the frontal lobe of the wearer.

2. The device of claim 1, wherein
the contact portions of the device are made of a flexible material to improve wearing comfort of the wearer; and
a vibration device 100 is provided inside the contact portions.

3. The device of claim 1, further comprising
a database configured to store sound sources.

4. The device of claim 1, wherein
the device communicates with an external device to receive sound sources.

5. The device of claim 3 or 4, wherein
the device
mixes 40 Hz vibration with the sound sources to create acoustic vibration for stimulation.

6. The device of claim 1, wherein
the vibration device 100 comprises:
a lower body having an open upper portion and forming an accommodation space therein; a lower bracket having open upper and lower portions and installed in the accommodation space of the lower body; a magnetic body fixedly installed on a lower surface of the lower body and generating a magnetic force; a bobbin installed inside the lower bracket above the magnetic body; a voice coil installed on an outer circumferential surface of the bobbin and interacting with the magnetic body; a cone damper installed on an upper surface of the bobbin, coupled to an upper surface of an edge of the lower bracket, and generating a vibration in a vertical direction by interaction between the magnetic body and the voice coil; a connecting member having a lower end coupled to a central portion of the cone damper and a central portion of the upper surface of the bobbin, and having a vibration probe for human body stimulation coupled to an upper end thereof, to transmit vibration generated from the cone damper to the vibration probe; an upper plate installed on an upper surface of the magnetic body and inducing a magnetic force of the magnetic body to be concentrated on the voice coil; an upper body having the connecting member inserted through a center of an upper surface thereof and covering the open upper portion of the lower body; and an upper bracket having an open lower surface, installed in an internal space of the upper body and coupled with the lower bracket, and having the connecting member inserted through an upper surface thereof.

7. The device of claim 6, wherein
the cone damper comprises:
a cone plate provided in a plate shape and generating vibration in a vertical direction; and a plurality of dampers extending in a radially curved band shape along an edge of the cone plate and having a coupling hole formed at an end thereof for screw coupling, wherein the dampers are fixedly coupled to an edge between the upper bracket and the lower bracket.

8. The device of claim 6, wherein
the connecting member is configured such that
an upper portion thereof is screw-coupled to the shaft of the vibration probe and a lower portion thereof is screw-coupled to the central portion of the cone damper and the central portion of the upper surface of the bobbin, wherein
the vibration probe is provided with a fixing groove along an outer circumferential surface of a specific position of the shaft; the connecting member has a coupling groove formed in the upper portion into which the shaft is inserted and removed, and includes an elastic fixing pin fixedly mounted to be seated in or detached from the fixing groove when the shaft is inserted into or separated from the coupling groove; and the lower portion is screw-coupled to the central portion of the cone damper and the central portion of the upper surface of the bobbin, and
the shaft includes a groove portion concave upward on one side where a bolt is not formed, and a vibrating body is coupled to the groove portion by a connecting body to swing in the groove portion,
thereby increasing vibration intensity of the vibration probe.
